# EUROPEAN PATENT APPLICATION

(11) **EP 1 870 094 A1**
(43) Date of publication of application: **26.12.2007**
(21) Application number: 06732070.5
(22) Date of filing: 12.04.2006
(51) Int. Cl.: A61K 31/00

(54) **ANTIWRINKLE AGENT**

(30) Priority: 13.04.2005 JP 2005116061
(71) Applicant: Shiseido Company, Limited, Chuo-ku Tokyo 104-8010 (JP)
(72) Inventor: TAKADA, Keiko, SHISEIDO RESEARCH CENTER, Yokohama-shi, Kanagawa 2248558 (JP); INOMATA, Shinji, SHISEIDO RESEARCH CENTER, Yokohama-shi, Kanagawa 2248558 (JP); OGURA, Yuki, SHISEIDO RESEARCH CENTER, Yokohama-shi, Kanagawa 2360004 (JP); UMISHIO, Kenichi, SHISEIDO RESEARCH CENTER, Yokohama-shi, Kanagawa 2248558 (JP)
(74) Representative: Santarelli
(86) International application number: PCT/JP2006/308163
(87) International publication number: WO 2006/109888

(57) **Abstract**

There is provided a novel anti-wrinkle composition comprising one or more from among watercress, juniper, baizhu and coltsfoot extracts as active ingredients.

## Description

### Technical Field

The present invention relates to a novel anti-wrinkle composition. More specifically, the invention relates to an anti-wrinkle composition whose active ingredient is an extract from watercress, juniper, baizhu, and/or coltsfoot. The invention further relates to a composition for stabilizing the basement membrane, a composition for promoting formation of extracellular matrix and a composition for improving skin sagging and tightness whose active ingredient is the aforementioned extract, and to a process for production of artificial skin, a method for reducing wrinkles and a method for improving skin sagging and tightness with use of the extract.

### Background Art

Skin, which covers the entirety of human and other animal bodies, is affected by several changes including wrinkle formation, hardening, spot formation, loss of color, reduced elasticity and the like as a result of external factors such as sunlight, dryness, oxidation, environmental stress and psychological stress, as well as aging. Skin is largely composed of two layers, the epidermis and the dermis. A thin, fine layer known as the basement membrane is found between the epidermis and dermis. Metabolism of the epidermis is dependent on a supply of blood and factors produced by dermal cells, through the basement membrane. Growth and differentiation of the skin epidermis is regulated by the basement membrane and dermis. Thus, communication between the epidermis and dermis via the basement membrane plays an important role in regulating the function of the skin epidermis.

The skin basement membrane has a special structure known as the anchoring complex, which performs the role of adhesion and stabilization of communication between the two tissues of the epidermis and dermis. The proteins of the anchoring complex are linked to both keratin, which forms the cytoskeleton of keratinocytes, and the connective tissue proteins of the dermal papillary layer. One of the important constituent elements of the anchoring complex is laminin 5 (Rousselle et al., J Cell Biol. 1991 Aug 114(3): 567-76). Research to date has demonstrated that genetic mutations in the genes coding for the three chains of laminin 5 are responsible for congenital genetic disorders characterized by formation of severe blistering, a condition known as Herlitz junctional epidermolysis bullosa (Aberdam et al., Nat Genet. 1994 Mar; 6(3): 299-304). This indicates that laminin 5 is an indispensable substance for epidermal adhesion. Laminin 5 forms anchoring filaments, and has been shown to bind with the integrin α6β4 (a transmembrane integrin found in the hemidesmosome), which is known to be a receptor for laminin 5 (Niessen et al., Exp Cell Res. 1994 Apr; 211(2): 360-7).

It has been reported that laminin 5 not only directly binds to type VII collagen which forms anchoring fibrils connected to the dermal papillary layer, but also forms complexes with laminin 6 or 7, the complexes in turn binding with type IV collagen as a skeleton of the basement membrane, via nidogen.

Lower expression levels of type IV collagen that forms the complexes are observed with age, (Vazquez F et al., Maturitas 1996, 25: 209-215), while reduced production of type VII collagen that binds with laminin 5 has been reported on both the protein level and mRNA level in skin fibroblasts from elderly individuals as compared to skin fibroblasts from young individuals (Chen et al., J. Invest. Dermatol., 102: 205-209, 1994). According to other reports, the anchoring fibrils composed of type VII collagen are reduced in number upon physiological aging or photoaging of normal skin (Tsuji, T., Nippi Kaishi, 105:963-975, 1995, Tidman et al., J. Invest Dermatol., 83: 448-453, 1984). In addition to the individual characteristics of the constituent components, the basement membrane itself is also known to exhibit structural abnormalities such as folded layering or rupturing with aging of the skin (Lavker et al., J. Invest. Dermatol. 1979 73: 59-66), and it is believed that such structural changes result in the signs of aging such as wrinkles and sagging and the reduced skin function associated with aging. It is believed, therefore, that promoting production of laminin 5 and type IV and type VII collagen, that are constituents of the basement membrane skeleton, is extremely important for maintaining a satisfactory basement membrane structure and preventing skin aging. In fact, reports of detailed observation of the skin basement membrane structure, showing frequent basement membrane damage during an individual's late 20s to early 30s, indicate that structural changes in the basement membrane play an important role in inducing skin aging (IFSCC Magazine, 2000 4(4): 15-23).

One major goal in preventing skin aging is to inhibit wrinkles. Wrinkles are largely classified as either "epidermal wrinkles" (fine wrinkles) that have shallow furrows or "dermal wrinkles" (large wrinkles) that have deep furrows, and for dermal wrinkles that result from changes in the basement membrane and degeneration or collapse of the dermis layer, it is believed that maintaining the basement membrane is especially important.

The aging dermis contains reduced amounts of not only the basement membrane constituents described above, but also type I and type III collagen that are abundantly present in the young dermis (approximately 80% and 15%, respectively), and it is considered highly likely that these changes are also responsible for reduced dermal elasticity and tightness, as well as wrinkles and dermal atrophy. Since the proportion of type III collagen, for example, is higher in infantile tissue from fetuses than in adult tissue, and type III collagen is not found in the tissue of type IV Ehlers-Danlos syndrome where wrinkles are prominent, type III collagen is most probably an important component for constructing normal soft skin.

Type I and type III collagens in the dermis are synthesized by fibroblasts. When fibroblasts are cultured in collagen gel, the collagen gel contracts.
The fiber density of the contracted collagen gel is similar to the fiber density in the dermis. This phenomenon occurs because fibroblasts attract collagen fibers. In actual skin as well, sufficient attraction of collagen fibers by fibroblasts is thought to maintain the tightness of the skin. It is therefore important to maintain not only the basement membrane but also the dermis in order to support homeostasis of the skin to prevent wrinkles and improve skin sagging and tightness.

From the standpoint of supporting homeostasis of the skin, there have in the past been proposed soybean-derived preparations, lysophosphatidylcholine, lysophosphatidic acid and the like, and extracts of plants belonging to the genera *Bacconia, Psophocarpus, Cassia* and *Erythraea,* aimed at reinforcing production of laminin 5, which has been established as an important component of the basement membrane (Japanese Unexamined Patent Publication No. 11-343226, Japanese Unexamined Patent Publication No. 2000-226308, Japanese Unexamined Patent Publication No. 2003-313135). Various plant-derived extracts such as extracts from soybean, beech germ, pueraria, English ivy and *Fagaceae Fagus* plants are also known to promote production of type III, type IV and type VII collagens (Japanese Unexamined Patent Publication No. 2001-39849, Japanese Unexamined Patent Publication No. 2004-18471, Japanese Unexamined Patent Publication No. 2004-75661). However, pueraria extract is the only known substance that promotes production of laminin 5 and type IV and type VII collagens which are important constituents of the basement membrane, as well as type III collagen which is an important constituent of the dermis (Japanese Unexamined Patent Publication No. 2001-39849, Japanese Unexamined Patent Publication No. 2003-137767, Japanese Unexamined Patent Publication No. 2004-18471).

### Detailed Description of the Invention

The present invention provides a novel anti-wrinkle composition.

As a result of much diligent research on various plant extracts to obtain anti-wrinkle effects particularly against wrinkles in the dermis, the present inventors have found that watercress, juniper, baizhu and coltsfoot significantly promote production of laminin 5 and type IV and type VII collagens which are important constituents of the basement membrane, as well as type III collagen which is an important constituent of the dermis, and that when applied onto the skin, they exhibit effects against skin aging and especially anti-wrinkle effects and improving effects on skin sagging and tightness. Furthermore, it was discovered that among these extracts, juniper, baizhu and coltsfoot also exhibit anti-inflammatory effects and wound healing effects. These plant extracts have not yet been reported to exhibit anti-wrinkle effects in aging skin, such as a promoting effect on production of extracellular matrix components including laminin 5.

Extract of watercress (*Nasturtium officinalis* R. Brown, a species of the Brassicaceae family) is used as a hair restorer because of its effects of improving blood circulation (Japanese Unexamined Patent Publication No. 2001-181120) and promoting expression of vascular endothelial cell growth factors (Japanese Unexamined Patent Publication No. 2003-313113). However, its effects on skin aging are unknown.

Extract of juniper (*Juniperus oxycedrus* Linne, a species of the Cupressaceae family) is known to have an effect of improving the skin barrier function which guards against skin roughening and the like (Japanese Unexamined Patent Publication No. 2001-288066), but its effects on skin aging, wrinkles, skin sagging and improved tightness are unknown. The improvement in the skin barrier function referred to here has an effect on the horny layer, but nothing is mentioned regarding the effect on the dermis under the horny layer.

Extract of baizhu (*Atractylodes macrocephale,* a species of the Compositae family) is known to have an effect against skin roughness (Japanese Unexamined Patent Publication No. 2003-194809) and an effect against hair graying (Japanese Unexamined Patent Publication No. 2004-161639), but its effects on skin aging, wrinkles, skin sagging and tightness are unknown.

Extract of coltsfoot (*Tussilago farfara* Linne, a species of the Asteraceae family), is known to have an effect against phototoxicity, including an effect of preventing aging induced by products of UVA irradiation on air pollutants (Japanese Unexamined Patent Publication No. 2002-12548), but its prevention and ameliorative effects on wrinkles caused by UVB irradiation and its effects on skin sagging and tightness improvement are unknown.

Therefore, the invention provides an anti-wrinkle composition, a composition for stabilizing the basement membrane, a composition for accelerating formation of extracellular matrix and a composition for improving skin sagging and tightness which comprises one or more active ingredients from among extracts of watercress, juniper, baizhu and coltsfoot, as well as a process for production of artificial skin and a cosmetic method for reduction of wrinkles and improvement of skin sagging and tightness, with use of the extract.

### Effect of the Invention

The anti-wrinkle composition of the invention comprises an extract of watercress, juniper, baizhu or coltsfoot, whereby it promotes formation of the extracellular matrix, including the components laminin 5, type IV collagen and type VII collagen which are important constituents of the basement membrane, and type III collagen which is an important constituent of the dermis. The accelerated extracellular matrix formation produces an anti-wrinkle effect by normalizing the condition of the skin and reducing the number and lengths of wrinkles via repair of the basement membrane and dermis, promotion of wound healing, reduction in transdermal transpiration of moisture and increased skin viscoelasticity. While the exact mechanism is not fully understood, it is believed that these effects are all the result of accelerated production of laminins and the like, which leads to repair and stabilization of the basement membrane and dermis. The aforementioned extracts can also help to improve the symptoms of epidermolysis bullosa, which results from destabilization of the basement membrane including lysis of basement membrane cells, separation of the basement membrane from the epidermis and abnormalities in type VII collagen. As regards the effect of the invention, it is to be noted that all of the aforementioned extracts accelerate production of not only one but all of laminin 5, type III, type IV collagen and type VII collagen.

### Best Mode for Carrying Out the Invention

### Anti-wrinkle composition

The anti-wrinkle composition of the invention comprises, as an active ingredient, an extract from watercress, juniper, baizhu and/or coltsfoot. The term "anti-wrinkle" as used herein means an "effect against skin aging" in the sense of preventing and/or improving skin wrinkles and especially reducing the number and lengths of skin wrinkles, and it may be used synonymously with "effect against skin aging". The "wrinkles" that are improved or prevented according to the invention are especially deep dermal wrinkles created by collapse of the dermis layer by changes in the basement membrane. These effects are judged by considering such factors as improved cuticle, increased skin moisture and increased skin elasticity, as determined by visual inspection, for example. The effects according to the invention are believed to be achieved because the extracts from watercress, juniper, baizhu and coltsfoot increase production of extracellular matrix components such as laminin 5, type III, type IV and type VII collagen, thereby stabilizing the basement membrane and dermis and improving homeostasis of the skin; however, the exact mechanism is not fully understood. Therefore, the anti-wrinkle effect obtained by the anti-wrinkle composition of the invention is, of course, not limited to increased production of extracellular matrix components. Coltsfoot extract is known to have a preventive/ameliorative effect on skin aging and wrinkle formation caused by UVA radiation (Japanese Unexamined Patent Publication No. 2002-12548), but its preventive or ameliorative effect on wrinkles induced mainly by UVB is unknown.

The term "effect against skin aging" as used throughout the present specification means preventing or improving reduced skin function, and specifically skin wrinkles, sagging and hardening, that occur when structural changes in the basement membrane accumulate with chronological aging or photoaging, in order to help maintain an elastic, youthful, healthy skin condition.

### Composition for stabilizing the basement membrane

The invention provides a composition for stabilizing the basement membrane comprising as an active ingredient one or more from among extracts of watercress, juniper, baizhu and coltsfoot. The concept of "stabilizing the basement membrane" as used herein means stabilization and repair of the skeleton of the basement membrane which is between the epidermis and dermis and is intimately involved in the maintenance of skin homeostasis. More specifically, the intended meaning is a general effect on the basement membrane brought about by accelerating production of components important for constructing the basement membrane, and particularly extracellular matrix components.

### Composition for promoting formation of the extracellular matrix

The invention further provides a composition for promoting formation of extracellular matrix comprising as an active ingredient one or more from among the aforementioned extracts. The term "extracellular matrix" as used throughout the present specification has the meaning generally recognized in the relevant field, and particularly it means the important constituents of the basement membrane including laminin 5 and type IV and type VII collagen, and the important constituents of the dermis such as type III collagen.

### Composition for improving skin sagging and tightness

The invention further provides a composition for improving skin sagging and tightness comprising as an active ingredient one or more from among the aforementioned extracts. The concept of "improving skin sagging and tightness" referred to throughout the present specification means improvement in tightness or sagging due to aging or external irritation such as drying and ultraviolet rays, by increasing the elasticity of or tightening the skin, and this may be evaluated, for example, *in vitro* based on the collagen gel contraction promoting effect, or in vivo based on the viscoelasticity of the skin.

The aforementioned extracts may exhibit, in addition to anti-wrinkle effects, basement membrane stabilizing effects, extracellular matrix formation promoting effects, skin sagging and tightness improving effects and collagen gel contraction promoting effects, also anti-inflammatory effects and wound healing effects. As was mentioned above, the mechanisms for such effects are not completely understood. The "anti-inflammatory effects" and "wound healing effects" mentioned throughout the present specification are determined in a relative manner as the effects with application of the plant extracts of the invention onto sites of inflammation and wound sites, as compared to areas without application of the same.
The wound healing effect obtained according to the invention is believed to occur because the basement membrane is stabilized and repaired as a result of accelerated formation of the extracellular matrix. However, the effect is not necessarily due to increased formation of the extracellular matrix. The watercress extract used for the invention has already been reported to have a wound healing effect (Japanese Unexamined Patent Publication No. 2003-313134).

In addition to the anti-inflammatory and wound healing effects described above, the plant extracts used for the invention also may exhibit an ameliorative effect on epidermolysis bullosa. The term "ameliorative effect on epidermolysis bullosa" as used throughout the present specification refers to not only to general alleviation and curing of epidermolysis bullosa which is classified as epidermolysis bullosa simplex, junctional epidermolysis bullosa or epidermolysis bullosa dystrophica, but also to prevention of its symptoms.
This effect is brought about by accelerated production of collagen or other important constituent components of the basement membrane, and stabilization of the basement membrane structure.

The plant extracts used for the invention are derived from watercress, juniper, baizhu and coltsfoot. For the herbaceous plants watercress, juniper, baizhu and coltsfoot, extraction is performed from the roots, leaves, stems and flowers, while for the arboreous plant juniper, extraction is performed from the roots, stems, bark, fruit, leaves and flowers.

The extraction process may be carried out by solvent extraction, in which case the plant material is dried if necessary and further shredded and crushed if necessary, and an aqueous extraction agent such as cold water, warm water or hot water at the boiling point or lower or an organic solvent such as methanol, ethanol, 1,3-butanediol or ether is used for extraction at ordinary temperature or with heating. However, the extraction process is not limited to solvent extraction and may be carried out by any ordinary method known in the field. The form of the extract does not have to be the extract itself, as it may be in a form obtained by appropriate dilution, concentration or drying of the stock by means of an ordinary method.

An anti-wrinkle composition, a composition for stabilizing basement membrane, a composition for promoting formation of extracellular matrix or a composition for improving skin sagging and tightness according to the invention may contain a single extract or a combination of more than one extract. In another embodiment of the invention, the anti-wrinkle composition, a composition for stabilizing basement membrane, a composition for promoting formation of extracellular matrix or a composition for improving skin sagging and tightness of the invention may further comprise a serine protease inhibitor. As described in the examples which follow, including a serine protease inhibitor in an anti-wrinkle composition, a composition for stabilizing basement membrane, a composition for promoting formation of extracellular matrix or a composition for improving skin sagging and tightness of the invention will inhibit decomposition of collagen and help maintain the basement membrane and dermis in a satisfactory condition. As s result, more satisfactory results may therefore be predicted for the anti-wrinkle, skin sagging and tightness improving, wound healing and epidermolysis bullosa improving effects that are obtained by the invention. The serine protease inhibitor include, but are limited to, extracts of peppermint, ichiyakuso, otogiriso, *Houttuyniae herba,* arnica, Chinese quince, hawthorn, meadowsweet, cabbage rose, multiflora rose, grape, peony, hop, raspberry and garden chamomile, which may be extracted by solvent extraction in the same manner as the extraction process for watercress and the like mentioned above. Other serine protease inhibitors include aprotinin, tranexamic acid, ε-aminocaproic acid and the like.

The extract used for the invention is added in an amount necessary to achieve an effect against skin aging and especially anti-wrinkle and skin sagging and tightness improving effects, or in an amount necessary to achieve stabilization of the basement membrane and dermis or increased production of extracellular matrix components such as laminin 5 and type III, type IV or type VII collagen. It may also be added in an amount necessary for the purpose of increasing the stratum corneum or transdermal moisture (ameliorating skin roughness), increasing skin elasticity or improving skin texture, which amount will vary depending on various factors including the form of addition. The extract may added at a concentration of 0.0001-10 wt% and preferably 0.001-1 wt% with respect to the entire anti-wrinkle composition, a composition for stabilizing basement membrane, a composition for promoting formation of extracellular matrix or a composition for improving skin sagging and tightness, although these ranges are not restrictive.

An anti-wrinkle composition, a composition for stabilizing basement membrane, a composition for promoting formation of extracellular matrix or a composition for improving skin sagging and tightness according to the invention may also contain, in addition to the extract(s) mentioned above, appropriate amounts of components approved for addition to cosmetics and drugs, such as liquid fats and oils, solid fats and oils, waxes, hydrocarbons, higher fatty acids, higher alcohols, esters, silicones, anionic surfactants, cationic surfactants, amphoteric surfactants, nonionic surfactants, humectants, water-soluble polymer compounds, thickening agents, coating agents, ultraviolet absorbers, metal ion blocking agents, lower alcohols, polyhydric alcohols, saccharides, amino acids, organic amines, pH regulators, skin nutrients, vitamins, antioxidants, aromatics, powders, coloring materials, water and the like, as necessary within ranges that do not interfere with the anti-wrinkle and other effects.

There may also be added in appropriate amounts L-ascorbic acid and its salts, L-ascorbic acid ester derivatives such as L-ascorbic acid phosphate esters, L-ascorbic acid sulfate esters and salts thereof, L-ascorbic acid glucosides such as L-ascorbic acid glucoside and salts thereof, alkoxysalicylic acids such as 4-methoxysalicylic acid and salts thereof, hydroquinone glucosides such as hydroquinone β-D-glucose, hydroquinone α-D-glucose and salts thereof, tranexamic acid, tranexamic acid derivatives such as methylamide tranexamate hydrochloride, resorcin derivatives such as 4-n-butylresorcin, kojic acid, ellagic acid, linolic acid, chamomile extract, retinoic acid, retinol, retinolacetic acid, retinolpalmitic acid, glycyrrhizic acid and its derivatives, laminin 5-production accelerators such as lysophosphatidylcholine or lysophosphatidic acid, soybean preparations, saccharides such as glucose, fructose, mannose, sucrose and trehalose, circulation improvers such as nicotinic acid benzyl ester, nicotinic acid β-butoxyethyl ester, capsaicin, zingerone, Cantharides tincture, ichthammol, caffeine, tannic acid, α-borneol, tocopherol nicotinate, inositol hexanicotinate, cyclandelate, cinnarizine, tolazoline, acetylcholine, verapamil, cepharanthin and y-oryzanol, antiseborrheic agents such as sulfur and thianthol, and any of various multipurpose compounds such as hinokitiol, iris extract, gambir extract, allantoin, aloe extract, ichiyakuso extract, garden thyme extract, turmeric extract, phellodendron bark extract, huang lian extract, otogiriso extract, restharrow extract, hydrolyzed casein, hydrolyzable yeast extract, pueraria extract, xylitol, kurara extract, rice extract hydrolysate, Bupleuri radix extract, saffron extract, zinc oxide, winged-bean extract, lithospermum root extract, Chinese peony extract, ginger extract, silica-coated zinc oxide, sage extract, mallow extract, senkyu extract, swertia extract, citrus unshiu extract, capsicum extract, toki extract, peach kernel extract, thiotaurine, ginseng extract, garlic extract, blackberry lily extract, hypotaurine, birch extract, loquat extract, grape extract, beech germ extract, luffa extract, marjoram extract, lily extract, coix extract, rosemary extract, amino acids and their derivatives including arginine and its hydrochloride and serine and its hydrochloride, pantothenic acid compounds such as calcium pantothenate, D-pantothenyl alcohol, pantothenyl ethyl ether and acetylpantothenyl ethyl ether, vitamin D compounds such as ergocalciferol and cholecalciferol, nicotinic acid compounds such as nicotinic acid, nicotinamide and benzyl nicotinate, vitamin E compounds such as α-tocopherol, tocopherol acetate, DL-α-tocopherol nicotinate and DL-α-tocopherol succinate, other vitamins such as vitamin K, vitamin P and biotin, and coenzymes such as ubiquinone.

The dosage form of the anti-wrinkle composition, a composition for stabilizing basement membrane, a composition for promoting formation of extracellular matrix or a composition for improving skin sagging and tightness of the invention is not particularly restricted, and for example, it may be in any desired form such as a solution system, a solubilized system, an emulsified system, a powder-dispersed system, a water-oil two-layer system, a water-oil-powder three-layer system, an ointment, gel, aerosol, or the like. The form of use is also not particularly restricted, and for example, any desired form such as lotion, emulsion, cream, essence, jelly, gel, ointment, pack, foundation or the like may be employed.

### External preparation for skin

The invention also provides an external preparation for skin comprising the aforementioned extract. The external preparation for skin according to the invention in this case is used for the purpose of providing an effect against skin aging (other than an anti-wrinkle effect or an effect of improving skin sagging and tightness), anti-inflammatory effect, wound healing effect and epidermolysis bullosa ameliorating effect. However, the external preparation for skin according to the invention is not limited to these purposes, and may be used for any improvement in skin symptoms by the extracellular matrix formation promoting effect of the extracts mentioned above.

The substances that may be added to the external preparation for skin of the invention are the same as for the anti-wrinkle composition mentioned above, and their types, amounts and concentrations may be determined by a person skilled in the art as appropriate for the desired purpose. For example, if the purpose is to produce an anti-inflammatory effect or wound healing effect, the extract may be added to a concentration of 0.0001-10 wt% and preferably 0.001-1 wt% based on the total weight of the external preparation for skin.

### Process for production of anti-wrinkle composition, a composition for stabilizing basement membrane, a composition for promoting formation of extracellular matrix or a composition for improving skin sagging and tightness

In another embodiment, the invention provides a process for production of an anti-wrinkle composition, a composition for stabilizing basement membrane, a composition for promoting formation of extracellular matrix or a composition for improving skin sagging and tightness, which comprises a step of adding an extract from watercress, juniper, baizhu or coltsfoot to an aqueous phase or oily phase. The process may also be applied to the external preparation for skin described above.

### Process for production of artificial skin

The invention still further provides a process for production of artificial skin characterized by adding one or more extracts of watercress, juniper, baizhu or coltsfoot to an artificial skin-forming medium.

The basal medium used to produce the artificial skin according to the invention may be any medium conventionally used for production of artificial skin, and as such media there may be mentioned Dulbecco's Modified Eagle's Medium (DMEM) containing 10% fetal calf serum; DMEM-Ham's F12(3:1) containing 10% fetal calf serum, 5 µg/ml transferrin, 5 µg/ml insulin, 2 nM triiodothyronine, 0.1 nM cholera toxin and 0.4 µg/ml hydrocortisone; and medium comprising keratinocyte growth medium (KGM) and 10% fetal calf serum-containing DMEM in a ratio of 1:1. The extract is added to the basal medium at about 0.001 wt%-1 wt%, depending on the type of extract. The different types of substances mentioned for use in the anti-wrinkle composition may also be added together with the extract(s), and the aforementioned serine protease inhibitors are particularly preferred because they inhibit collagen decomposition and stabilize the basement membrane.

For production of artificial skin according to the invention, contracted type I collagen gel containing human fibroblasts is first situated on a wire gauze. The contracted type I collagen gel containing the human fibroblasts can be prepared in the following manner, for example. A fibroblast-suspending collagen solution is prepared on ice, and then the collagen is gelled in a petri dish. The gel is then released from the petri dish walls and the collagen gel is contracted in a CO₂ incubator.

Next, epidermal cells (normal human epidermal keratinocytes) are cultured on the collagen gel to form an epidermis. Formation of an epidermal layer by culturing of skin cells may be accomplished in the following manner. Contracted collagen gel is placed on wire gauze and a glass ring is situated on the gel. A human prepuce epidermal keratinocyte suspension is placed in the glass ring while avoiding liquid leakage. The keratinocytes are adhered in a CO₂ incubator and the ring is removed. The medium is filled to the border of the epidermal layer and culturing is continued while exposing the epidermal layer to air, to form a stratum corneum.

This method produces artificial skin similar to natural skin, having basement membrane components adequately deposited between an epidermal layer and a dermis layer composed of a fibroblast-containing contracted type I collagen gel. The extract used for the invention may be added not only to fresh medium when the medium is exchanged at a point several days after seeding of the epidermal cells, but also as necessary at any other stage.

### Wrinkle-reducing method and skin sagging and tightness improving method

In another embodiment, the invention provides a cosmetic method characterized by reducing wrinkles and improving skin sagging and tightness by applying one or more extracts from among watercress, juniper, baizhu and coltsfoot to skin. The method is intended not only to reduce wrinkles and improve skin sagging and tightness, but also to promote extracellular matrix formation and ameliorate skin symptoms such as epidermolysis bullosa, which requires stabilization of the basement membrane and dermis. According to this method, it is assumed that the extract is applied to a mammal, and especially to a human.

### EXAMPLES

The present invention will now be explained in greater detail by the following examples.

### (Experiment method-1)

### Test method for laminin 5 production accelerating effect

### (1) Culturing of epidermal cornified cells

Epidermal cornified cells were separated from human prepuce and cultured in an epidermal cell growth medium (KGM) with a low calcium concentration. Bovine pituitary extract and EGF were added to the medium. The cells were cultured in KGM to the 4th generation, the adhering cells were suspended by trypsin-EDTA treatment, and cell aggregates were removed by filtration to obtain a homogeneous cell suspension. The cells were collected by centrifugal separation and resuspended in DMEM-F12 (2:1)-0.1% BSA to 8 × 10⁴/ml. A 0.5 ml portion of the cell suspension was added to 0.5 ml of the same medium containing a two-fold concentration of the active agent. Culturing was continued at 37°C for 24 hours using a 24-well plate. Upon completion of culturing, the culture supernatant was transferred to an Eppendorf tube, centrifugal separation was performed at 10,000 rpm for 5 minutes, and the supernatant was transferred to a new tube and stored at -20°C until measurement of laminin 5. In order to solubilize laminin 5 bound to the intracellular portion thereof and onto the culturing plastic, Tris-HCl buffers (pH 7.4) containing different surfactants were added to each well and the mixtures were stored overnight at -20°C. On the following day, ultrasonic treatment was performed and the mixtures were refrozen. After relysis on the next day, centrifugal separation was performed at 10,000 rpm for 5 minutes and the supernatants were transferred to tubes and stored at -20°C until measurement of laminin 5.

### (2) Measurement of laminin 5 by sandwich ELISA

Laminin 5 in the culture supernatants and cell layer was measured by sandwich ELISA. Monoclonal antibody BM165 for the laminin α3 chain of laminin 5 was bound to the solid layer of a 96-well ELISA plate. For assay of laminin 5 by the sandwich method, monoclonal antibody 6F12 for the laminin β3 chain was pre-biotinylated (b-6F12) for use as a different antibody. This method measures only the functionable heterotrimer (α3β3γ2), but detects no heterodimer (β3γ2). Sample was added to each well that already contained 3% gelatin/phosphate buffer with b-6F12. The final dilution rate of sample in the wells was 1/4 in the culture solution and 1/10 in the cell layer. Antigen-antibody reaction was conducted at 37°C for 2 hours, and after rinsing the plate, avidin-HRP (horseradish peroxidase) solution was added and reaction was continued from 30 minutes to 1 hour. After rinsing, ABTS was added as HRP substrate and the absorbance at 405 nm was measured with an ELISA plate reader. A calibration curve was drawn for the range of 0-40 ng/ml.

Production of laminin 5 was calculated as the sum of the amount released into the medium and the amount remaining in the cell layer, and it was expressed as a value relative to a sample without addition of plant extract (control). A summary of the increase in laminin 5 production for each extract is shown in Table 1 below.

**Table 1**

| (Results-1) | | |
|---|---|---|
| Samples | Concentration (%) | Laminin 5 production with respect to extract-free control (%) |
| Japanese | 0.001 | 125 |
| Watercress | 0.01 | 135 |
| Juniper | 0.001 | 117 |
| | 0.01 | 120 |
| Baizhu | 0.001 | 115 |
| | 0.01 | 129 |
| Japanese | 0.001 | 107 |
| Coltsfoot | 0.01 | 110 |

### (Experiment method-2)

### Test method for type IV collagen and type VII collagen production accelerating effect

### (1) Culturing of human fibroblasts

Human fibroblasts cultured in 10% FBS-containing DMEM were seeded in a 24-well plate, and after cell adhesion, the medium was exchanged with 0.25% FBS- and 250 µM ascorbic acid glucoside-containing DMEM and the active agent was added. After one day, the culture supernatant was collected and centrifuged, and then the type IV and type VII collagen in the supernatant was measured, and the amount of DNA in the cells was measured as an index of the cell count.

### (2) DNA quantitation

Measurement of DNA was accomplished by fluorometry using H33342 by Hoechst.

### (3) Measurement of type IV and type VII collagen by sandwich ELISA

Type IV and type VII collagen was measured by the sandwich ELISA method. The following antibodies were used in this example.
· Type IV collagen-specific antibodies: monoclonal antibody JK-199 and polyclonal antibody MO-S-CLIV
· Type VII collagen-specific antibodies: monoclonal antibody NP-185 and monoclonal antibody NP-32

The amounts of type IV and type VII collagen with respect to DNA in the active agent-added samples was compared to the amounts of type IV and type VII collagen with respect to DNA in a sample without active agent addition (control) defined as 100, to determine the type IV and type VII collagen production acceleration rate. The collagen production acceleration ratios are summarized in Table 2 below.

**Table 2**

| (Results-2) | | | | |
|---|---|---|---|---|
| | | 0.005% | 0.05% | 0.5% |
| Watercress | Type IV | 144 | 176 | 159 |
| | Type VII | 109 | 127 | 132 |
| Juniper Baizhu | Type IV | 107 | 114 | 120 |
| | Type VII | 101 | 105 | 107 |
| | Type IV | 124 | 135 | 140 |
| | Type VII | 107 | 110 | 114 |
| Coltsfoot | Type IV | 113 | 117 | 130 |
| | Type VII | 107 | 125 | 129 |

### (Experiment method-3)

### Test method for type III collagen production accelerating effect

### (1) Culturing of human skin fibroblasts

Human skin fibroblasts cultured in 10% FBS-containing DMEM were seeded in a 24-well plate, and after adhesion of the cells, the medium was exchanged with 0.25% FBS- and 250 µM magnesium ascorbate phosphate-containing DMEM, and the active agent was added. After 3 days, the culture supernatant was collected and centrifuged. The amount of type III collagen obtained in the supernatant was measured, while the cellular DNA was also assayed and used as an index of the cell count.

### (2) DNA quantitation

Measurement of DNA was accomplished by fluorometry using H33342 by Hoechst.

### (3) Measurement of type III collagen

Type III collagen biosynthesis by the cells was evaluated by assaying the amount of type III collagen terminal peptide (Procollagen type III-peptide: PIIIP) secreted in the culture supernatant. Specifically, a "Riagnost PIIIP assay kit" (CIS Diagnostics) was used for the assay. The amount of type III collagen production was divided by the amount of DNA and expressed as the relative value with respect to a sample without addition of a plant extract (control) (see Table 3).

**Table 3**

| (Results-3) | | |
|---|---|---|
| Sample | Concentration (%) | Type III collagen production (%) with respect to DNA, relative to non-added control |
| Watercress extract | 0.001 | 117 |
| | 0.01 | 124 |

These results indicated a significant type III collagen production accelerating effect for watercress extract.

### (Experiment method-4)

### Method of evaluating effect against type I collagen gel contraction by human skin fibroblasts

A fibroblast (5 × 10⁴ cell/ml)-suspended collagen solution (using type I collagen by Kouken Co., Ltd.) was prepared on ice and the collagen was gelled at 37°C.
Next, DMEM containing the test substance (DMSO was used as a control) and 0.25% FBS was added, the gel was released from the plate walls and the collagen was contracted (number of groups n = 3). The collagen gel diameter was measured from three directions after one and two days, and the average value was determined. The contraction ratio after addition of the test substance was determined with the diameter before contraction defined as a contraction ratio of 0% (see Table 4).

**Table 4**

| (Results-4) | | |
|---|---|---|
| Control (DMSO) | 1 day | 12% |
| | 2 days | 21% |
| Watercress extract (0.001%) | 1 day | 18% |
| | 2 days | 27% |
| Watercress extract (0.003%) | 1 day | 25% |
| | 2 days | 34% |

These results indicated a significant collagen gel contraction accelerating effect for watercress extract.

### (Experiment method-5)

### Process for production of artificial skin

Collagen gel was produced by preparing a human dermis-derived fibroblast (1 × 10⁵ cells/ml)-suspended collagen solution on ice and then gelling the collagen at 37°C in a 60 mm petri dish. The collagen gel was then released from the plate and placed on metal, and then a glass ring (inner diameter: 12 mm) was placed over the gel. A KGM-DMEM (1:1) mixed medium containing a human prepuce epidermal keratinocyte suspension was then added to the glass ring while avoiding liquid leakage. The epidermal cells were incubated overnight for adhesion and the ring was removed on the following day. The medium was filled to the border of the epidermal layer and the epidermal layer was exposed to air to prepare a skin model having a layered epidermis exhibiting stratum corneum formation.

From the 4th day after seeding of the epidermal cells, the medium was exchanged with medium containing (1) no active agent, (2) 0.3% watercress extract, (3) 0.3% watercress extract + 0.5% peppermint extract, (4) 0.3% juniper oil, (5) 0.3% juniper oil + 0.5% ichiyakuso extract, (6) 0.3% baizhu extract, (7) 0.3% baizhu extract + 0.5% otogiriso extract, (8) 0.3% coltsfoot extract or (9) 0.3% coltsfoot extract + 0.5% Houttuyniae herba extract, after which it was exchanged with medium containing the same type and concentration of active agent every 2-3 days, and culturing was continued for two weeks.

The formed artificial skin was haematoxylin-eosin stained and then immunostained (anti-type IV collagen antibody and anti-type VII collagen antibody). The extent of staining of the type IV and type VII collagen was scored on a 5-level scale, with 1 at the lowest and 5 at the highest. The scores for the extent of staining of collagen are summarized in Table 5 below.

**Table 5**

| (Results-5) | | |
|---|---|---|
| | Extent of staining of type IV collagen | Extent of staining of type VII collagen |
| (1) | 2 | 1 |
| (2) | 5 | 4 |
| (3) | 5 | 5 |
| (4) | 3 | 3 |
| (5) | 4 | 5 |
| (6) | 4 | 3 |
| (7) | 5 | 5 |
| (8) | 3 | 3 |
| (9) | 4 | 4 |

Thus, type IV collagen was weakly stained in the control type (1), while almost no type VII collagen was observed. In contrast, it was confirmed in this artificial skin that watercress, juniper, baizhu and coltsfoot, which have laminin 5/type IV collagen/type VII collagen production accelerating effects, all increase the extent of staining of both type IV and type VII collagen. Each extract of the serine protease inhibitors peppermint, ichiyakuso, otogiriso and *Houttuyniae herba* were shown to further increase the extents of staining.

### (Experiment method-6)

Extracts of watercress, juniper, baizhu and coltsfoot were applied onto the skin of three healthy males aged 30-45, and the stratum corneum moisture was measured and compared with a control area. Ion-exchanged water was used as a control. Each specimen was applied onto a 2.5 cm square within the inner forearm, and the stratum corneum moisture was measured just before application and one hour thereafter using a Corneometer CM825C (Courage+Khazaka), to determine the moisture increase rate. The stratum corneum moisture increase rates with application of each plant extract are summarized in Table 6 below.

**Table 6**

| (Results-6) | | | | |
|---|---|---|---|---|
| Stratum corneum moisture increase rates 1 hour after application of test substances | | | | |
| Control area | Active agent-applied area | | | |
| | Watercress | Juniper | Baizhu | Coltsfoot |
| 1% | 40% | 35% | 38% | 30% |

This demonstrated that application of the test substances increases moisture in the stratum corneum and improves the skin.

### (Experiment method-7)

A prolonged administration test was conducted by a panel of healthy women aged 40-55, using creams with the following formulations. A panel of 48 women was divided into 4 groups, and one of different creams (invention products 1-4) were applied onto half of the faces in each group while a comparison product 1 was applied onto the other half, every day twice a day for 4 weeks. The stratum corneum moisture was measured with a Corneometer CM825C, the transdermal moisture transpiration was measured with a Tewameter TM210 and the skin viscoelasticity was measured with a Cutometer (all products of Courage+Khazaka), as indices of the moisture increase, skin roughness improvement and tightness improvement, respectively. With the Cutometer, pulling for 2 seconds and opening for 1 second was repeated 3 times in the ordinary manner, and comparison was made between the skin elongations at the first and third times (Uf value, maximal amplitude) and the viscosity/elasticity (Uv/Ue value) with respect to the value recorded before prolonged administration. A replica was also taken of the eye corner using a Silicone Replica (Silflo, Flexico Developments), and the number and lengths of wrinkles were measured and compared with the values before prolonged administration. Also, an expert panel visually judged the skin texture condition and uniformity of stratum corneum sampling, after taking a sample of the stratum corneum with tape.

The results (comparison with values before prolonged administration) are shown in Tables 7-11. The numerical values in parentheses are test values obtained by a paired student t-test.

**Table 7**

| (Results-7) | | | | |
|---|---|---|---|---|
| · Analysis results using replica | | | | |
| | Invention product 1 group | Invention product 2 group | Invention product 3 group | Invention product 4 group |
| Number of wrinkles | -9% (p = 0.003) | -6% (p = 0.004) | -7% (p = 0.009) | -6% (p = 0.003) |
| | | | | |
| Lengths of wrinkles | -12% (p = 0.010) | -10% (p = 0.009) | -11% (p = 0.015) | -9% (p = 0.008) |

These results showed a statistically significant anti-wrinkle effect by the invention products.

**Table 8**

| · Stratum corneum moisture | | | | |
|---|---|---|---|---|
| | Invention product 1 group | Invention product 2 group | Invention product 3 group | Invention product 4 group |
| | +22% (p = 0.0001) | +24% (p = 0.0001) | +20% (p = 0.0001) | +15% (p = 0.0008) |

These results showed a statistically significant moisture retention effect by the invention products.

**Table 9**

| · Epidermal moisture transpiration | | | | |
|---|---|---|---|---|
| | Invention product 1 group | Invention product 2 group | Invention product 3 group | Invention product 4 group |
| | -40% (p = 0.0001) | -34% (p = 0.0009) | -33% (p = 0.0009) | -27% (p = 0.0012) |

These results showed a statistically significant skin roughness improving effect by the invention products.

**Table 10**

| · Skin viscoelasticity | | | | |
|---|---|---|---|---|
| | Invention product 1 group | Invention product 2 group | Invention product 3 group | Invention product 4 group |
| Uf(1st time) | -20% (p = 0.0052) | -21% (p = 0.0049) | -19% (p = 0.0073) | -23% (p = 0.0051) |
| | | | | |
| Uf(3rd time) | -21% (p = 0.0007) | -29% (p = 0.0033) | -25% (p = 0.0019) | -24% (p = 0.0088) |
| | | | | |
| Uv/Ue | -25% (p = 0.0030) | -22% (p = 0.0002) | -30% (p = 0.0007) | -18% (p = 0.0001) |

These results showed a significant skin elasticity increasing effect by the invention products.

**Table 11**

| · Texture | | | | |
|---|---|---|---|---|
| | Invention product 1 group | Invention product 2 group | Invention product 3 group | Invention product 4 group |
| Texture condition | +23% (p = 0.0021) | +20% (p = 0.0088) | +33% (p = 0.0071) | +18% (p = 0.0009) |
| | | | | |
| Uniformity of stratum corneum sampling | +33% (p = 0.0001) | +34% (p = 0.0022) | +40% (p = 0.0005) | +29% (p = 0.0002) |

These results showed a significant texture improving effect by the invention products.

The compositions of the invention products 1-4 and the comparison product are shown in Table 12 below. The numerical values are expressed as weight percentages with respect to the total.

**Table 12**

| | Invention products | | | | Comparison product 1 |
|---|---|---|---|---|---|
| | 1 | 2 | 3 | 4 | |
| Sample | | | | | |
| Watercress extract | 1.0 | - | - | - | - |
| Juniper oil | - | 1.0 | - | - | - |
| Baizhu extract | - | - | 1.0 | - | - |
| Coltsfoot extract | - | - | - | 1.0 | - |
| Glycerin | 10.0 | 10.0 | 10.0 | 10.0 | 10.0 |
| 1,3-Butylene glycol | 4.0 | 4.0 | 4.0 | 4.0 | 4.0 |
| Ethanol | 7.0 | 7.0 | 7.0 | 7.0 | 7.0 |
| Polyoxyethylene (20 mol) oleyl alcohol | 0.5 | 0.5 | 0.5 | 0.5 | 0.5 |
| Purified water | remainder | remainder | remainder | remainder | remainder |

### (Experiment method-8)

### Anti-inflammation and wound healing test

Inflammation or wounding was artificially produced in 8-week-old HR-1 mice, and 0.5 g of 1,3-butylene glycol containing (1) no active agent, (2) 5% juniper oil, (3) 5% baizhu extract or (4) 5% coltsfoot extract was applied to each group (n = 5) twice a day for 5 days, after which the conditions of the inflamed or wounded sites were observed on the 5th day. The anti-inflammatory and wound healing effects were scored on a 5-level scale with 1 as the lowest and 5 as the highest. The results are summarized in Table 13.

**Table 13**

| (Results-8) | | |
|---|---|---|
| | Anti-inflammatory effect | Wound healing effect |
| (1) | 1 | 2 |
| (2) | 3 | 4 |
| (3) | 4 | 4 |
| (4) | 5 | 4 |

As shown in Table 13, the degree of improvement in inflammation and wound healing was lower with the control (1). In contrast, the juniper, baizhu and coltsfoot extracts were all confirmed *in vivo* to have anti-inflammatory and wound healing effects.

### (Example 1 - Cream)

### (1) Formulation

| (Phase A) | |
|---|---|
| Stearic acid | 10.0 wt% |
| Stearyl alcohol | 4.0 |
| Butyl stearate | 8.0 |
| Monoglycerin stearate ester | 2.0 |
| Vitamin E acetate | 0.5 |
| Vitamin A palmitate | 0.1 |
| Macadamia nut oil | 1.0 |
| Preservative agent | q.s. |
| Perfume | q.s. |

| (Phase B) | |
|---|---|
| Glycerin | 4.0 |
| 1,2-Pentanediol | 3.0 |
| Potassium hydroxide | 0.4 |
| Magnesium ascorbate phosphate | 0.1 |
| L-Arginine hydrochloride | 0.01 |
| Trisodium edetate | 0.05 |
| Watercress 1,3-butylene glycol extract | 0.5 |
| Ion-exchanged water | q.s. |

### (2) Preparation method

The oily phase A and aqueous phase B were each heated to 70°C to complete dissolution. Phase A was added to phase B and the mixture was emulsified with an emulsifier. The emulsion was cooled using a heat exchanger.

### (Example 2 - Cream)

### (1) Formulation

| | |
|---|---|
| Stearic acid | 5.0 wt% |
| Stearyl alcohol | 4.0 |
| Isopropyl myristate | 18.0 |
| Glycerin monostearic acid ester | 3.0 |
| Propylene glycol | 10.0 |
| Juniper methanol extract | 0.01 |
| Caustic potash | 0.2 |
| Sodium hydrogen sulfite | 0.01 |
| Preservative agent | q.s. |
| Perfume | q.s. |
| Ion-exchanged water | q.s. |

### (2) Preparation method

The propylene glycol, juniper methanol extract and caustic potash were added to and dissolved in the ion-exchanged water, and the mixture was heated and kept at 70°C (aqueous phase). The other components were also combined and the mixture was heated to dissolution and kept at 70°C (oily phase). The oily phase was slowly added to the aqueous phase, and reaction was conducted a short while after completing the addition, while maintaining the temperature. The mixture was then homogeneously emulsified with a homomixer and cooled to 30°C while thoroughly stirring.

### (Example 3 - Wrinkle preventing cream)

### (1) Formulation

| | |
|---|---|
| Stearic acid | 2.0 wt% |
| Stearyl alcohol | 7.0 |
| Hydrogenated lanolin | 2.0 |
| Squalane | 5.0 |
| 2-Octyldodecyl alcohol | 6.0 |
| Polyoxyethylene (25 mol) cetyl alcohol ether | 3.0 |
| Glycerin monostearic acid ester | 2.0 |
| Propylene glycol | 5.0 |
| Baizhu ethanol extract | 0.05 |
| Turmeric ethanol extraction | 0.05 |
| Sodium hydrogen sulfite | 0.03 |
| Ethylparaben | 0.3 |
| Perfume | q.s. |
| Ion-exchanged water | q.s. |

### (2) Preparation method

The propylene glycol was added to the ion-exchanged water, and the mixture was heated and kept at 70°C (aqueous phase). The other components were mixed, heated to dissolution and kept at 70°C (oily phase). The oily phase was added to the aqueous phase and pre-emulsified, and after homogeneously emulsifying with a homomixer, the mixture was cooled to 30°C while mixing well.

### (Example 4 - Antiaging cream)

### (1) Formulation

| | |
|---|---|
| Solid paraffin | 5.0 wt% |
| Beeswax | 10.0 |
| Vaseline | 15.0 |
| Liquid paraffin | 41.0 |
| Glycerin monostearic acid ester | 2.0 |
| Polyoxyethylene (20 mol) sorbitan monolauric acid ester | 2.0 |
| Soap powder | 0.1 |
| Borax | 0.2 |
| Coltsfoot acetone extract | 0.05 |
| Bupleurum root ethanol extraction | 0.05 |
| Sodium hydrogen sulfite | 0.03 |
| Ethylparaben | 0.3 |
| Perfume | q.s. |
| Ion-exchanged water | q.s. |

### (2) Preparation method

The soap powder and borax were added to the ion-exchanged water, and the mixture was heated to dissolution and kept at 70°C (aqueous phase). The other components were combined, heated to dissolution and kept at 70°C (oily phase). The oily phase was slowly added to the aqueous phase while stirring, for reaction. Upon completion of the reaction, the mixture was homogeneously emulsified with a homomixer, and then thoroughly stirred while cooling to 30°C.

### (Example 5 - Emulsion)

### (1) Formulation

| | |
|---|---|
| Stearic acid | 1.0 wt% |
| Vaseline | 5.0 |
| Liquid paraffin | 10.0 |
| Polyoxyethylene (10 mol) | |
| monooleic acid ester | 2.0 |
| Polyethylene glycol 1500 | 3.0 |
| Triethanolamine | 1.0 |
| Carboxyvinyl polymer (trade name: CARBOPOL 941, B.F. Goodrich Chemical company) | 0.2 |
| Watercress ethyl acetate extract | 0.01 |
| Sodium hydrogen sulfite | 0.01 |
| Ethylparaben | 0.3 |
| Perfume | q.s. |
| Ion-exchanged water | q.s. |

### (2) Preparation method

The carboxyvinyl polymer was dissolved in a small amount of the ion-exchanged water (phase A). The polyethylene glycol 1500 and triethanolamine were added to the remaining ion-exchanged water, and the mixture was heated to dissolution and kept at 70°C (aqueous phase). The other components were combined, heated to dissolution and kept at 70°C (oily phase). The oily phase was added to the aqueous phase for pre-emulsification, and then phase A was added and the mixture was homogeneously emulsified with a homomixer and cooled to 30°C while thoroughly stirring.

### (Example 6 - Emulsion)

### (1) Formulation

| | |
|---|---|
| Microcrystalline wax | 1.0 wt% |
| Beeswax | 2.0 |
| Lanolin | 2.0 |
| Liquid paraffin | 10.0 |
| Squalane | 5.0 |
| Sorbitan sesquioleic acid ester | 4.0 |
| Polyoxyethylene (20 mol) sorbitan monooleic acid ester | 1.0 |
| Propylene glycol | 7.0 |
| Juniper 1,3-butylene glycol extract | 10.0 |
| Sodium hydrogen sulfite | 0.01 |
| Ethylparaben | 0.3 |
| Perfume | q.s. |
| Ion-exchanged water | q.s. |

### (2) Preparation method

The propylene glycol was added to the ion-exchanged water, and the mixture was heated and kept at 70°C (aqueous phase). The other components were combined, heated to dissolution and kept at 70°C (oily phase). The oily phase was stirred while the aqueous phase was slowly added thereto, and the mixture was homogeneously emulsified with a homomixer. The emulsion was cooled to 30°C while thoroughly stirring.

### (Example 7 - Emulsion)

### (1) Formulation

| (Phase A) | |
|---|---|
| Squalane | 5.0 wt% |
| Oleyl oleate | 3.0 |
| Vaseline | 2.0 |
| Sorbitan sesquioleic acid ester | 0.8 |
| Polyoxyethylene oleyl ether (2OEO) | 1.2 |
| Evening primrose oil | 0.5 |
| Preservative agent | q.s. |
| Perfume | q.s. |

| (Phase B) | |
|---|---|
| 1,3-Butylene glycol | 4.5 |
| Baizhu ethanol extract | 1.5 |
| Ethanol | 3.0 |
| Carboxyvinyl polymer | 0.2 |
| Potassium hydroxide | 0.1 |
| L-Arginine L-aspartic acid salt | 0.01 |
| Pueraria ethanol extract | 1.5 |
| Erythritol | 0.5 |
| Hexasodium metaphosphate | 0.05 |
| Ion-exchanged water | q.s. |

### (2) Preparation method

The oily phase A and aqueous phase B were each heated to 70°C to complete dissolution. Phase A was added to phase B and the mixture was emulsified with an emulsifier. The emulsion was cooled using a heat exchanger.

### (Example 8 - Jelly)

### (1) Formulation

| | |
|---|---|
| 95% Ethyl alcohol | 10.0 wt% |
| Dipropylene glycol 1 | 15.0 |
| Polyoxyethylene (50 mol) oleyl alcohol ether | 2.0 |
| Carboxyvinyl polymer (trade name: CARBOPOL 940, B.F. Goodrich Chemical company) | 1.0 |
| Caustic soda | 0.15 |
| L-Arginine | 0.1 |
| Coltsfoot ethanol extraction | 7.0 |
| Sodium 2-hydroxy-4-methoxybenzophenonesulfonate | 0.05 |
| Trisodium ethylenediaminetetraacetate 2H₂O | 0.05 |
| Methylparaben | 0.2 |
| Perfume | q.s. |
| Ion-exchanged water | q.s. |

### (2) Preparation method

After uniformly dissolving CARBOPOL 940 in ion-exchanged water, coltsfoot ethanol extract and polyoxyethylene (50 mol) oleyl alcohol ether were dissolved in 95% ethanol and added to the aqueous phase. After then adding the other components, the mixture was neutralized and thickened with the caustic soda and L-arginine.

### (Example 9 - Essence)

### (1) Formulation

| (Phase A) | |
|---|---|
| Ethyl alcohol (95%) | 10.0 wt% |
| Polyoxyethylene (20 mol) octyldodecanol | 1.0 |
| Pantothenyl ethyl ether | 0.1 |
| Watercress methanol extract | 1.5 |
| Methylparaben | 0.15 |

| (Phase B) | |
|---|---|
| Potassium hydroxide | 0.1 |

| (Phase C) | |
|---|---|
| Glycerin | 5.0 |
| Dipropylene glycol | 10.0 |
| Sodium hydrogen sulfite | 0.03 |
| Carboxyvinyl polymer (trade name: CARBOPOL 940, B.F. Goodrich Chemical company) | 0.2 |
| Ion-exchanged water | q.s. |

### (2) Preparation method

Phase A and phase C were each uniformly dissolved, and then phase A was added to phase C for solubilization. After then adding phase B, the mixture was packed.

### (Example 10 - Pack)

### (1) Formulation

| (Phase A) | |
|---|---|
| Dipropylene glycol | 5.0 wt% |
| Polyoxyethylene (60 mol) hydrogenated castor oil | 5.0 |

| (Phase B) | |
|---|---|
| Juniper extract | 0.01 |
| Olive oil | 5.0 |
| Tocopherol acetate | 0.2 |
| Ethylparaben | 0.2 |
| Perfume | 0.2 |

| (Phase C) | |
|---|---|
| Sodium hydrogensulfite | 0.03 |
| Polyvinyl alcohol (saponification degree: 90, polymerization degree: 2, 000) | 13.0 |
| Ethanol | 7.0 |
| Ion-exchanged water | q.s. |

### (2) Preparation method

Phase A, phase B and phase C were each uniformly dissolved, and phase B was added to phase A for solubilization. After then adding phase C, the mixture was packed.

### (Example 11 - Lotion)

### (1) Formulation

| (Phase A) | |
|---|---|
| Ethanol | 5.0 wt% |
| POE oleyl alcohol ether | 2.0 |
| Oleyl alcohol | 0.1 |
| 2-Ethylhexyl-P-dimethyl aminobenzoate | 0.18 |
| Perfume | q.s. |

| (Phase B) | |
|---|---|
| 1,3-Butylene glycol | 9.5 |
| Glycerin | 2.0 |
| Sodium pyrrolidonecarboxylate | 0.5 |
| Nicotinamide | 0.3 |
| Baizhu 1,3-butyleneglycol extract | 0.1 |
| β-Cyclodextrin | 1.0 |
| Erythritol | 0.05 |
| Ion-exchanged water | q.s. |

### (2) Preparation method

The alcohol phase A was added to the aqueous phase B for solubilization to obtain lotion.

### (Example 12 - Solid foundation)

### (1) Formulation

| | |
|---|---|
| Talc | 43.1 wt% |
| Kaolin | 15.0 |
| Sericite | 10.0 |
| Zinc flower | 7.0 |
| Titanium dioxide | 3.8 |
| Yellow iron oxide | 2.9 |
| Black iron oxide | 0.2 |
| Squalane | 8.0 |
| Isostearic acid | 4.0 |
| POE sorbitan monooleate | 3.0 |
| Isocetyl octanoate | 2.0 |
| Coltsfoot ethanol extract | 0.5 |
| Preservative agent | q.s. |
| Perfume | q.s. |

### (2) Preparation method

The talc-black iron oxide powder components were thoroughly mixed with a blender, and then the squalane-isocetyl octanoate oily components, coltsfoot ethanol extract, preservative agent and perfume were added and thoroughly kneaded therewith, after which the mixture was packed and molded in a container.

### (Example 13 - Emulsified foundation (cream type))

### (1) Formulation

| (Powder portion) | |
|---|---|
| Titanium dioxide | 10.3 wt% |
| Sericite | 5.4 |
| Kaolin | 3.0 |
| Yellow iron oxide | 0.8 |
| Red iron oxide | 0.3 |
| Black iron oxide | 0.2 |

| (Oily phase) | |
|---|---|
| Decamethylcyclopentasiloxane | 11.5 |
| Liquid paraffin | 4.5 |
| Polyoxyethylene-modified dimethylpolysiloxane | 4.0 |

| (Aqueous phase) | |
|---|---|
| Ion-exchanged water | 50.0 |
| 1,3-Butylene glycol | 4.5 |
| Watercress ethanol extract | 1.5 |
| Sorbitan sesquioleic acid ester | 3.0 |
| Preservative agent | q.s. |
| Perfume | q.s. |

### (2) Preparation method

After heating and stirring the aqueous phase, the thoroughly mixed and pulverized powder portion was added and the mixture was treated with a homomixer. The heated and mixed oily phase was added, the mixture was treated with a homomixer, and then the perfume was added while stirring and the mixture was cooled to room temperature.

## Claims

1. An anti-wrinkle compositioin comprising one or more from among watercress, juniper, baizhu and coltsfoot extracts as active ingredients.

2. A composition for stabilizing basement membrane comprising one or more from among watercress, juniper, baizhu and coltsfoot extracts as active ingredients.

3. A composition for promoting formation of extracellular matrix comprising one or more from among watercress, juniper, baizhu and coltsfoot extracts as active ingredients.

4. A composition for promoting formation of extracellular matrix according to claim 3, wherein the constituent components of the extracellular matrix whose formation is to be promoted are one or more components selected from the group consisting of laminin 5, type III collagen, type IV collagen and type VII collagen.

5. A composition for promoting formation of extracellular matrix according to claim 3 or 4, **characterized in that** formation of all of the components laminin 5, type III collagen, type IV collagen and type VII collagen is promoted.

6. A composition for improving skin sagging and tightness comprising one or more from among watercress, juniper, baizhu and coltsfoot extracts as active ingredients.

7. A process for production of artificial skin, comprising a step of adding one or more from among watercress, juniper, baizhu and coltsfoot extracts to artificial skin-forming medium.

8. A cosmetic method **characterized by** reducing wrinkles and improving skin sagging and tightness by applying one or more from among watercress, juniper, baizhu and coltsfoot extracts to skin.
